(19) Europäisches Patentamt / European Patent Office / Office européen des brevets

(11) **EP 3 621 089 B1**

(12) **EUROPÄISCHE PATENTSCHRIFT**

(45) Veröffentlichungstag und Bekanntmachung des Hinweises auf die Patenterteilung:
**25.08.2021 Patentblatt 2021/34**

(51) Int Cl.:
*H01F 1/00* (2006.01)      *A61K 6/16* (2020.01)
*A61K 6/71* (2020.01)      *A61K 6/84* (2020.01)

(21) Anmeldenummer: **18193477.9**

(22) Anmeldetag: **10.09.2018**

(54) **DENTALMATERIAL MIT MAGNETPARTIKELN MIT VERBESSERTER FARBABSCHIRMUNG**

DENTAL MATERIAL WITH MAGNETIC PARTICLES WITH IMPROVED COLOUR SHIELDING

MATÉRIAU DENTAIRE AVEC PARTICULES À ÉCRAN COULEUR AMÉLIORÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**11.03.2020 Patentblatt 2020/11**

(73) Patentinhaber: **Ivoclar Vivadent AG**
**9494 Schaan (LI)**

(72) Erfinder:
• **Rist, Kai**
**6800 Feldkirch (AT)**
• **Schmidt, Annette**
**50733 Köln (DE)**
• **Shaaban, Ahmad**
**50825 Köln (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
EP-A1- 0 959 107      EP-A2- 0 609 897
WO-A2-2010/149150      DE-T5-112012 005 417
JP-B2- 3 032 927      US-A1- 2014 371 341

**Beschreibung**

Die vorliegende Erfindung betrifft Dentalmaterialien, insbesondere zur Herstellung von Adhäsiven und Zementen.

Magnetische Partikel und insbesondere magnetische Nanopartikel, d.h. magnetische Teilchen mit einem Durchmesser von unter 300 nm, sind auf Grund ihres großen Anwendungspotentials in verschiedenen Bereichen wie unter anderem Katalyse, Diagnostik, Therapie, Wasserbehandlung und Datenspeicherung von großem Interesse.

Zum Schutz oder zur Stabilisierung der Partikel, z.B. gegen Korrosion oder Agglomeration, ist es bekannt, eine Beschichtung auf die Partikel aufzubringen. Eine Beschichtung kann auch zur weiteren Funktionalisierung oder Modifizierung der Partikel für weitere verschiedene Anwendungen dienen. Beispielsweise beschreibt die DE 10 2015 118 816 anisotrope Eisenoxidnanopartikel mit einem Durchmesser von 3 bis 50 nm, die in eine silikatische Matrix eingebettet sind und als Bestandteil von Sicherheitsmerkmalen z.B. in Dokumenten oder Wertpapieren verwendet werden können. Nanokristalline magnetische Eisenoxidpartikel, die von einem Hüllmaterial aus natürlichen oder synthetischen Glycosaminoglycanen umgeben sind, und deren Anwendung in der medizinischen Diagnostik und Therapie werden beispielsweise in der DE 41 17 782 offenbart. Ferner beschreibt die EP 1 894 214 ein Verfahren zur Herstellung von Silicabeschichteten Magnetpartikeln und deren Verwendung zur Aufreinigung von Nukleinsäuren aus biologischen Körperproben.

Des Weiteren ist es bekannt, dass magnetische Partikel grundsätzlich zur lokalen indirekten Wärmeerzeugung genutzt werden können. So können Magnetpartikel, die einem elektromagnetischen Wechselfeld ausgesetzt werden, auf Grund von Hysterese und Relaxationsprozessen ein Teil der Energie des Wechselfelds in Wärme umwandeln. Eine beispielhafte Anwendung unter Ausnutzung dieses Effekts stellt die Thermobehandlung von Tumorzellen mit ferngesteuerter induktiver Erwärmung im Radiofrequenzbereich dar, wobei die Tumorzellen durch lokale Hitzeeinwirkung abgetötet werden.

Die Fähigkeit zur induktiven Wärmeerzeugung von Magnetpartikeln kann auch zum gezielten Lösen von Klebeverbindungen, dem sog. Debonding-on-Demand (DoD), genutzt werden. Dabei kann das Lösen (debond) von Klebeverbindungen gezielt (on demand) dadurch erreicht werden, dass die Festigkeit der adhäsiven Verbundschicht durch Erwärmung deutlich verringert wird. So schlägt beispielsweise die WO 2013/034777 den Einbau von ferromagnetischen Nanopartikeln in Dentalmaterialien, insbesondere Adhäsiven und Zementen, vor, um durch Einwirkung eines magnetischen Wechselfeldes eine induktive Erwärmung des Dentalwerkstoffs zu erreichen. Im Dentalbereich ist das Lösen von Klebeverbindungen unter anderem in der Kieferorthopädie von Bedeutung, wo Brackets, die zur Korrektur von Zahnfehlstellungen auf die Zahnoberfläche geklebt werden, nach erfolgter Korrektur ohne Schädigung des Zahnschmelz wieder entfernen werden müssen. Außerdem wären im Falle der Reparatur oder des vollkommenen Ersatzes von hochfesten keramischen Restaurationen oder Kronen, die mechanisch nur aufwändig entfernbar sind, einfach erweichbare bzw. trennbare Zementverbunde von Vorteil.

Ein Nachteil des Einsatzes von Magnetpartikeln in Dentalmaterialien stellt jedoch häufig die dunkle Farbe der Magnetpartikel dar. Insbesondere Teilchen, deren Durchmesser groß genug ist, um maßgeschneiderte magnetische Eigenschaften für Debonding-on-Demand-Anwendungen bereitzustellen, wie z.B. eine ausreichende Koerzitivität, sind für das menschliche Auge als grauer oder dunkler Punkt im Dentalmaterial sichtbar oder führen insgesamt zu einer Verdunklung des Dentalmaterials, was aus ästhetischen Gründen unerwünscht ist.

Zwar ist bekannt, die Farbe von magnetischen Teilchen durch Zugabe von weißen Pigmenten aufzuhellen. Beispielsweise beschreibt die JP 2000/150218 die Beschichtung von Magnetkernen mit weißen Pigmenten. Die JP 2003/002658 offenbart weiße Magnetteilchen, die dadurch erhalten werden, dass feine magnetische Eisenoxidteilchen auf die Oberfläche von weißen $TiO_2$-Kernen aufgebracht werden. Ferner beschreiben die US 5,194,356 und die WO 2010/149150 jeweils den Einbau von magnetischen Teilchen und weißen Pigmenten in eine Polymermatrix.

DE 11 2012 005417, JP 3 032927, EP 0 959 107 und EP 0 609 897 beschreiben Magnetpartikel mit mehreren Hüllen und ansteigendem Brechungsindex.

Allerdings haben die vorliegenden Erfinder gefunden, dass Magnetpartikel, die mit Pigmenten aufgehellt sind, wie z.B. Eisenoxidpartikel mit einer silicahaltigen Beschichtung, und an Luft eine nahezu neutrale Farbe mit nur leicht hellgrauem Ton aufweisen, bei Einbettung in einem Dentalmedium, wie z.B. Zahnzement, eine deutliche dunklere Farbe zeigen.

Der Erfindung liegt somit die Aufgabe zugrunde, Partikel mit verbesserter Farbabschirmung bereitzustellen. Insbesondere liegt der Erfindung die Aufgabe zugrunde, Magnetpartikel bereitzustellen, die ausreichende magnetische Eigenschaften für die lokale, induzierte Wärmeerzeugung aufweisen und sich somit für Debonding-on-Demand-Anwendungen im Dentalbereich eignen und gleichzeitig in Adhäsivmedien, wie Dentaladhäsiven oder Zahnzementen, ein ästhetisches Erscheinungsbild, d.h. eine möglichst neutrale Farbe, aufweisen.

Diese Aufgabe wird erfindungsgemäß durch den polymerisierbaren Dentalwerkstoff des Anspruchs 1 mit Magnetpartikeln gelöst, die einen magnetischen Kern und eine erste Beschichtung aus erstem Hüllmaterial umfassen, wobei auf der vom Kern wegweisenden Oberfläche der ersten Beschichtung eine zweite Beschichtung aus zweitem Hüllmaterial aufgebracht ist, wobei das zweite Hüllmaterial von dem ersten Hüllmaterial verschieden ist und einen höheren Brechungsindex als das erste Hüllmaterial aufweist.

Es wurde überraschend gefunden, dass durch die erfindungsgemäße Kombination einer ersten und zweiten Beschichtung eine unerwünschte Farbe des Partikelkerns, insbesondere die unerwünschte dunkle Farbe von Magnetpartikel, nicht nur an Luft, sondern auch in Adhäsivmedien, wie Dentaladhäsiven oder Zahnzementen, wirksam abgeschirmt werden kann. Somit zeichnen sich die erfindungsgemäßen Magnetpartikel nicht nur durch ausreichende magnetische Eigenschaften für die lokale, induzierte Wärmeerzeugung, sondern insbesondere auch durch hervorragende optische Eigenschaften bei Einbettung in Adhäsivmedien aus. Ohne an irgendeine Theorie gebunden sein zu wollen, wird angenommen, dass bei erfindungsgemäßen Partikeln eine unerwünschte dunkle Farbe, d.h. Absorption von einfallendem Licht durch das Kernmaterial, dadurch minimiert wird, dass einfallendes Licht durch das erfindungsgemäße Verhältnis der Brechungsindices der Hüllmaterialien vom Kern weg gelenkt wird und die Doppelhülle somit zu einer Fokussierung des einfallenden Lichts außerhalb des Kerns führt. Die erfindungsgemäße Beschichtung von magnetischen Kernen kann somit auch bei Einbettung der Partikel in Medien mit einem Brechungsindex von größer als 1,0, wie z.B. 1,4 bis 1,5, wie er üblicherweise bei Adhäsivmedien im Dentalbereich auftritt, zu einer wirksamen Farbabschirmung führen.

Um die gewünschte Farbabschirmung möglichst effektiv zu erreichen, sind in einer Ausführungsform sowohl die erste als auch die zweite Beschichtung als kontinuierliche Beschichtung ausgebildet. Besonders bevorzugt ist die Oberfläche des Kerns vollständig vom ersten Hüllmaterial bedeckt und die vom Kern wegweisende Oberfläche der ersten Beschichtung ist vollständig vom zweiten Hüllmaterial bedeckt. Ferner ist es bevorzugt, dass sowohl die erste als auch die zweite Beschichtung im Wesentlichen gleichmäßig ausgebildet sind, d.h. dass sowohl die Dicke der ersten Beschichtung als auch die Dicke der zweiten Beschichtung über ihre gesamte Erstreckung im Wesentlichen konstant ist.

In einer bevorzugten Ausführungsform, bei der die gewünschte Farbabschirmung des Kerns besonders wirksam ist, beträgt das Verhältnis des Brechungsindex des zweiten Hüllmaterials zu dem Brechungsindex des ersten Hüllmaterials 1,1 bis 2,6, vorzugsweise 1,4 bis 2,0, besonders bevorzugt 1,6 bis 1,8.

In einer Ausführungsform weist das erste Hüllmaterial einen Brechungsindex von 1,1 bis 2,5, vorzugsweise 1,2 bis 1,9, besonders bevorzugt 1,3 bis 1,7 auf. Ferner weist das zweite Hüllmaterial vorzugsweise einen Brechungsindex von 1,7 bis 3,1, insbesondere 2,1 bis 3,1, besonders bevorzugt 2,5 bis 3,1 auf.

Soweit nichts anderes angegeben ist, bezieht sich die Angabe des Brechungsindex des ersten und zweiten Hüllmaterials in der vorliegenden Anmeldung auf die Stoffkonstante des jeweiligen Materials bei einer Wellenlänge von 589 nm (Natrium-D-Linie) und einer Temperatur von 25°C.

In einer weiteren Ausführungsform ist es bevorzugt, dass das Verhältnis der Dicke der zweiten Beschichtung zu der Dicke der ersten Beschichtung 0,1 bis 2,5, vorzugsweise 0,5 bis 2,0, besonders bevorzugt 1,0 bis 2,0 beträgt. Insbesondere beträgt die Dicke der ersten Beschichtung vorzugsweise 50 bis 1000 nm, besonders bevorzugt 100 bis 600 nm. Ferner beträgt die Dicke der zweiten Beschichtung vorzugsweise 50 bis 1000 nm, besonders bevorzugt 100 bis 600 nm.

Dabei wird die Dicke der ersten und zweiten Beschichtung mit Hilfe von Rasterelektronenmikroskopie (REM) bestimmt, wobei vom zahlenmittleren Durchmesser der Partikel mit zu bestimmender Beschichtung der zahlenmittlere Durchmesser der Partikel ohne zu bestimmender Beschichtung abgezogen wird. Die Dicke der Beschichtung ist gleich die Hälfte dieser Differenz der Durchmesser, d.h. die Dicke der Beschichtung entspricht der Differenz der entsprechenden Radien.

Ferner ist es bevorzugt, dass das Verhältnis der Dicke der zweiten Beschichtung zu dem Radius der ersten Beschichtung 0,1 bis 1, vorzugsweise 0,2 bis 0,8, besonders bevorzugt 0,28 bis 0,8 beträgt.

Dabei wird der Radius der ersten Beschichtung insbesondere mit Hilfe von Rasterelektronenmikroskopie (REM) bestimmt, wobei der mit der ersten Beschichtung beschichtete Kern vorzugsweise eine im Wesentlichen sphärische Gestalt aufweist und der Radius der ersten Beschichtung somit die Hälfte des zahlenmittleren Durchmessers der mit erster Beschichtung versehenen Kerne beträgt.

Besonders bevorzugt ist das zweite Hüllmaterial ausgewählt aus der Gruppe bestehend aus $TiO_2$, $ZrO_2$, ZnS, $Al_2O_3$, Poly(pentabromphenylmethacrylat) und Mischungen davon. Weiterhin ist das erste Hüllmaterial besonders bevorzugt ausgewählt aus der Gruppe bestehend aus $SiO_2$, $SiO_2$-Acrylat, Polystyrol, Polymethylmethacrylat, Polyvinylalkohol, Polyalkylacrylat, Polycarbonat, $MgF_2$, fluoriertem Polymer, $Al_2O_3$, $ZnO_2$ und Mischungen davon.. Dabei bezeichnet der Begriff "$SiO_2$-Acrylat" Polymere von 3-(Trimethoxysilyl)propylmethacrylat (TPM). Beispiele für geeignete fluorierte Polymere umfassen fluorierte Acrylate mit niedrigem Brechungsindex, z.B. Poly(1,1,1,3,3,3-hexafluorisopropylacrylat), Poly(2,2,3,3,4,4,4-heptafluor-butylacrylate), Poly(2,2,3,3,4,4,4-heptafluorbutylmeth-acrylat), Poly(2,2,3,3,3-pentafluorpropylacrylat), Poly(1,1,1,3,3,3-hexafluorisopropylmethacrylat), Poly(2,2,3,4,4,4-hexafluorbutylacrylat), Poly(2,2,3,4,4,4-hexafluorbutylmethacrylat), Poly(2,2,3,3,3-pentafluor-propylacrylat), Poly(2,2,2-trifluorethylacrylat), Poly(2,2,3,3-tetrafluorpropylacrylat), Poly(2,2,3,3-tetrafluorpropylmethacrylat) und Poly(2,2,2-trifluor-ethylmethacrylat).

Ganz besonders bevorzugt sind Magnetpartikel, bei denen das erste Hüllmaterial $SiO_2$ und das zweite Hüllmaterial $TiO_2$ ist. Weiterhin ist das $SiO_2$ des ersten Hüllmaterials vorzugsweise poröses und insbesondere mikroporöses

SiO$_2$.

Der Kern der erfindungsgemäßen Partikel ist vorzugsweise nicht vollständig lichtdurchlässig, sondern opak. Insbesondere im Bereich des sichtbaren Lichts weist der Kern eine Trübung oder anderweitig unerwünschte Farbe auf, wodurch sich der Wunsch nach einer Farbabschirmung ergibt.

Wie oben erwähnt, ist der Kern der erfindungsgemäßen Partikel magnetisch, so dass die erfindungsgemäßen Partikel Magnetpartikel darstellen. Der magnetische Kern solcher erfindungsgemäßen Magnetpartikel ist induktiv aufheizbar. Insbesondere wandelt der Kern elektromagnetische Energie in thermische Energie um, wenn er einem elektromagnetischen Wechselfeld ausgesetzt wird. In einer Ausführungsform ist der magnetische Kern ferromagnetisch oder ferrimagnetisch.

In einer weiteren Ausführungsform umfasst der magnetische Kern ein Metalloxid ausgewählt aus der Gruppe bestehend aus (i) Eisenoxid, insbesondere Hämatit (Fe$_2$O$_3$) und/oder Magnetit (Fe$_3$O$_4$), (ii) Ferriten der allgemeinen Formel MeO·Fe$_2$O$_3$, wobei Me ausgewählt ist aus der Gruppe bestehend aus Ni, Zn, Mn, Co, Cu, Mg, Cd, Ba und Sr, (iii) Mischferriten der allgemeinen Formel Me$_y$Fe$_{3-y}$O$_4$, wobei Me ausgewählt ist aus der Gruppe bestehend aus Ni, Zn, Mn, Co, Cu, Mg, Cd, Ba und Sr und y zwischen 0 und 1 beträgt, (iv) Metallen und Metalllegierungen aus der Gruppe bestehend aus Co, Ni, Fe, Nd, AlNiCo, FeNi, SmCo und NdFeB und (v) weiteren Oxiden wie z.B. CrO$_2$. Bevorzugt ist der magnetische Kern ausgewählt aus der Gruppe bestehend aus Fe$_3$O$_4$, CoFe$_2$O$_4$ und Co$_y$Fe$_{3-y}$O$_4$, wobei y zwischen 0 und 0,3 beträgt. Besonders bevorzugt besteht der magnetische Kern aus Fe$_3$O$_4$.

Der magnetische Kern kann eindomänig oder mehrdomänig sein. Vorzugsweise enthält ein erfindungsgemäßer Magnetpartikel einen mehrdomänigen Kern. Ferner weist der magnetische Kern vorzugsweise einen mittleren Durchmesser von 10 bis 1000 nm, insbesondere 25 bis 700 nm, besonders bevorzugt 100 bis 500 nm auf, wobei der mittlere Durchmesser der mittels Transmissionselektronenmikroskopie (TEM) bestimmte zahlenmittlerer Durchmesser ist.

Geeignete magnetische Kerne sind beispielsweise unter dem Handelsnamen Bayoxide® der Fa. Lanxess erhältlich.

Der magnetische Kern kann kugelförmig, würfelförmig oder stäbchenförmig sein. Vorzugsweise weist der Kern eine im Wesentlichen kugelförmige oder würfelförmige Gestalt auf.

Demgemäß weist auch der erfindungsgemäße beschichte Partikel eine kugelförmige, würfelförmige oder stäbchenförmige und vorzugsweise eine im Wesentlichen kugelförmige Gestalt auf. In einer bevorzugten Ausführungsform weist der erfindungsgemäße Partikel einen mittleren Durchmesser von 10 bis 2000 nm, vorzugsweise 25 bis 1500 nm, besonders bevorzugt 300 bis 1000 nm auf, wobei der mittlere Durchmesser der zahlenmittlere Durchmesser ist, der besonders bevorzugt mittels REM bestimmt wird, wobei für kleinere Partikel, wie Partikel mit einem Durchmesser von z.B. weniger als 400 nm, auch eine Bestimmung mittels TEM möglich ist.

Die erfindungsgemäßen Partikel lassen sich einfach herstellen. Beispielsweise können die Partikel, und insbesondere die erfindungsgemäßen Magnetpartikel, durch ein Verfahren hergestellt werden, bei dem

(a) magnetische Kerne dispergiert werden,
(b) eine Vorläuferverbindung des ersten Hüllmaterials zu der in Schritt (a) erhaltenen Dispersion gegeben wird und die erhaltene Mischung Bedingungen ausgesetzt wird, die ausreichen, um das erste Hüllmaterial als erste Beschichtung auf den Kernen abzuscheiden,
(c) eine Dispersion der mit dem ersten Hüllmaterial beschichteten Kerne bereitgestellt wird und
(d) eine Vorläuferverbindung des zweiten Hüllmaterials zu der in Schritt (c) erhaltenen Dispersion gegeben wird und die erhaltene Mischung Bedingungen ausgesetzt wird, die ausreichen, um das zweite Hüllmaterial als zweite Beschichtung auf der ersten Beschichtung abzuscheiden.

In Schritt (a) wird eine Dispersion von Kernen, insbesondere magnetischen Kernen, bereitgestellt. Vorzugsweise werden die Kerne mit Hilfe von Ultraschall oder eines geeigneten Rührers in einem geeigneten Lösungsmittel dispergiert. Vorzugsweise wird ein alkoholisches Lösungsmittel, insbesondere Ethanol, verwendet.

In Schritt (b) werden die Kerne mit dem ersten Hüllmaterial beschichtet. Dazu wird zu der Dispersion der Kerne eine Vorläuferverbindung des ersten Hüllmaterials gegeben. Als Vorläuferverbindung eignen sich beispielsweise metallorganische Vorläuferverbindungen, wie Alkoxymetallverbindungen. Im Falle von SiO$_2$ als erstem Hüllmaterial wird vorzugsweise ein Alkoxysilan, insbesondere Tetraethylorthosilicat, als Vorläufermaterial zugegeben. Die Abscheidung des ersten Hüllmaterials kann durch Zugabe eines Katalysators, wie z.B. Säuren oder Basen, begünstigt werden. Beispielsweise kann im Fall von Alkoxysilanen als Vorläufermaterial eine Base wie NH$_4$OH als Katalysator zugegeben werden. Die Zugabe des Katalysators kann vor der Zugabe, gleichzeitig mit der Zugabe oder nach der Zugabe der Vorläuferverbindung erfolgen. Besonders bevorzugt wird NH$_4$OH vor der Zugabe des SiO$_2$-Vorläufermaterials zugegeben.

Die Beschichtung in Schritt (b) erfolgt vorzugsweise bei Raumtemperatur. Des Weiteren ist es bevorzugt, dass die Reaktionsmischung in Schritt (b) während des Beschichtungsvorgangs weiter dispergiert wird, z.B. mittels Ultraschall oder intensivem Rühren. Optional können die mit der ersten Beschichtung versehenen Partikel abgetrennt werden, z.B.

mit Hilfe von magnetischen Verfahren, und ggf. charakterisiert werden.

Im Anschluss daran wird in Schritt (c) eine Dispersion der einfach beschichteten Kerne bereitgestellt. Vorzugsweise werden auch die beschichteten Kerne mit Hilfe von Ultraschall oder eines geeigneten Rührers in einem geeigneten Lösungsmittel, wie einem alkoholischen Lösungsmittel, dispergiert. Im Fall von $SiO_2$ als erstem Hüllmaterial werden die einfach beschichteten Partikel vorzugsweise in einer Mischung von THF und Ethanol dispergiert.

In Schritt (d) wird zu der Dispersion eine Vorläuferverbindung des zweiten Hüllmaterials gegeben. Geeignete Vorläuferverbindungen umfassen beispielsweise metallorganische Vorläuferverbindungen, wie Alkoxymetallverbindungen. Im Falle von $TiO_2$ als zweitem Hüllmaterial wird vorzugsweise ein Titanat, insbesondere Tetrabutylorthotitanat, als Vorläufermaterial zugegeben. Die Abscheidung des zweiten Hüllmaterials kann durch Zugabe eines Katalysators, wie z.B. Säuren oder Basen, begünstigt werden. Beispielsweise kann im Fall von Titanaten als Vorläufermaterial eine Base wie $NH_4OH$ als Katalysator zugegeben werden. Die Zugabe des Katalysators kann vor der Zugabe, gleichzeitig mit der Zugabe oder nach der Zugabe der Vorläuferverbindung erfolgen. Besonders bevorzugt wird $NH_4OH$ nach der Zugabe des $TiO_2$-Vorläufermaterials, insbesondere 30 bis 120 min und bevorzugt 40 bis 90 min nach der Zugabe des Vorläufermaterials, zugegeben. Es wurde gefunden, dass durch eine derartige Zugabe die Porosität der zweiten Beschichtung verringert werden kann.

Vorzugsweise wird die Beschichtung in Schritt (d) bei einer Vorläuferkonzentration von 0,10 bis 0,40 mol/L, insbesondere 0,15 bis 0,30 mol/L, besonders bevorzugt 0,17 bis 0,29 mol/L, durchgeführt. Außerdem ist es bevorzugt, dass die Katalysatorkonzentration 0,005 bis 0,03 mol/L, insbesondere 0,01 bis 0,015 mol/L, beträgt. Es wurde gefunden, dass insbesondere bei der Verwendung von Titanaten, wie Tetrabutylorthotitanat, als Vorläuferverbindung und der Verwendung einer Base, wie $NH_4OH$, als Katalysator die obigen bevorzugten Konzentrationsbereiche zu einer gleichmäßigen und dichten Beschichtung mit verringerter Porosität und Rissbildung führen.

Die erfindungsgemäßen Magnetpartikel können nach Schritt (d) abgetrennt werden, z.B. mit Hilfe von magnetischen Verfahren, und ggf. charakterisiert werden.

Die erfindungsgemäßen Magnetpartikel können insbesondere als Füllstoff für die lokale Wärmeerzeugung verwendet werden. Insbesondere sind die erfindungsgemäßen Magnetpartikel aufgrund des magnetischen Kerns in der Lage, lokal Wärme zu erzeugen, wenn sie einem elektromagnetischen Wechselfeld ausgesetzt werden. Die lokal erzeugte Wärme kann beispielsweise zum gezielten Lösen von Adhäsivverbindungen genutzt werden. Somit können die erfindungsgemäßen Magnetpartikel besonders gut als Füllstoff mit Debonding-on-Demand-Eigenschaften verwendet werden.

Gegenstand der Erfindung ist somit auch die Verwendung der erfindungsgemäßen Magnetpartikel als Füllstoff für die lokale Wärmeerzeugung in Dentalwerkstoffen wie im Anspruch 10, insbesondere als Adhäsivmaterialien mit Debonding-on-Demand-Eigenschaften.

Des Weiteren erfüllen die erfindungsgemäßen Magnetpartikel aufgrund der wirksamen Farbabschirmung der an sich dunklen und opaken Magnetkerne auch die ästhetischen Anforderungen, die an Dentalmaterialien gestellt werden. Somit können die erfindungsgemäßen Magnetpartikel insbesondere auch als Füllstoff in Dentalmaterialien verwendet werden. Mithin betrifft die vorliegende Erfindung auch die Verwendung der erfindungsgemäßen Magnetpartikel als Füllstoff in Dentalmaterialien, insbesondere Adhäsiven und Zementen.

Die Dentaladhäsive oder Zahnzemente können insbesondere zur reversiblen Befestigung beispielsweise von Brackets, Kronen oder Verblendungen (Veneers) verwendet werden. Dabei erfolgt vorzugsweise zunächst die Bildung eines Verbundes durch Aushärtung von Materialien (Adhäsiv oder Zement) auf Basis von z.B. einer oder mehrerer thermolabilen polymerisierbaren Verbindungen. Zum Debonding müssen die geklebten Teile kurzzeitig auf eine Temperatur erwärmt werden, die über der Temperatur liegt, bei der die Spaltung der thermolabilen Bindungen einsetzt. Dabei kann eine gezielte Energiezufuhr mittels induktiver Erwärmung der erfindungsgemäßen Magnetpartikel durch Einwirkung eines magnetischen Wechselfeldes erreicht werden.

Somit betrifft die Erfindung einen polymerisierbaren Dentalwerkstoff, der neben einer polymerisierbaren Verbindung erfindungsgemäße Magnetpartikel enthält. Die polymerisierbare Verbindung ist vorzugsweise ein Monomer mit Debonding-on-Demand-Eigenschaften, d.h. ein Monomer, das zur Bildung einer Adhäsivschicht führt, deren Festigkeit z.B. durch Erwärmung deutlich verringert werden kann. Derartige Monomere mit Debonding-on-Demand-Eigenschaften werden beispielsweise in der WO 2013/034777 und der WO 2016/005540 beschrieben.

Wie oben beschrieben, führt die Doppelhüllenbeschichtung der erfindungsgemäßen Magnetpartikel überraschenderweise dazu, dass die Partikel nicht nur an Luft, sondern auch in Dentalmedien, wie z.B. Dentaladhäsiven und Zahnzementen, eine hellere Farbe aufweisen. Mithin betrifft die vorliegende Erfindung auch die Verwendung einer ersten Beschichtung aus erstem Hüllmaterial und einer zweiten Beschichtung aus zweitem Hüllmaterial zur Farbmaskierung von Partikeln, insbesondere Magnetpartikeln, wobei die erste Beschichtung auf dem zu maskierenden Partikel und die zweite Beschichtung auf der vom Partikel wegweisenden Oberfläche der ersten Beschichtung aufgebracht ist und das zweite Hüllmaterial von dem ersten Hüllmaterial verschieden ist und einen höheren Brechungsindex als das erste Hüllmaterial aufweist.

Im Folgenden wird die Erfindung anhand von Ausführungsbeispielen näher erläutert.

**Ausführungsbeispiele**

**Beispiel 1**

Herstellung von Fe$_3$O$_4$-Kernen mit Doppelhüllenbeschichtung

In diesem Beispiel wird die Herstellung von Fe$_3$O$_4$-Kernen mit einer Doppelhüllenbeschichtung beschrieben, wobei die innere Beschichtung SiO$_2$ und die äußere Beschichtung TiO$_2$ ist. Derartig beschichtete Partikel werden im Folgenden auch als TiO$_2$@SiO$_2$@Fe$_3$O$_4$ bezeichnet. Die Herstellung erfolgte in zwei Schritten. Im ersten Schritt wurden magnetische Fe$_3$O$_4$-Partikel mit SiO$_2$ beschichtet. Nachfolgend wurden die erhaltenen SiO$_2$@Fe$_3$O$_4$-Partikel mit TiO$_2$ beschichtet.

*i) Herstellung von SiO$_2$@Fe$_3$O$_4$:*

Magnetische Fe$_3$O$_4$-Partikel (würfelförmig; zahlenmittlerer Durchmesser gemäß TEM von 226 $\pm$ 77 nm) wurden in einem 500 mL Dreihalsrundkolben mit Hilfe eines Ultraschallbads für 5 min in Ethanol (EtOH) dispergiert. Ammoniak (NH$_4$OH, 25%) und Wasser wurden dazugegeben und für weitere 5 min dispergiert. Tetraethylorthosilicat (TEOS) wurde in 100 mL EtOH verdünnt und unter Schutzgas (N$_2$) bei Raumtemperatur zu der Partikeldispersion getropft. Nach der Zugabe von TEOS wurde die Partikeldispersion für 1h im Ultraschallbad weiter dispergiert. Anschließend wurden die Partikel mit EtOH gereinigt, magnetisch getrennt, unter Vakuum getrocknet und der magnetische Gehalt mit Hilfe von Vibrationsmagnetometrie (*Vibrating Sample Magnetometry, VSM*) bestimmt.

Die eingesetzten Mengen an Magnetpartikel, TEOS, EtOH und NH$_4$OH zur Herstellung von SiO$_2$@Fe$_3$O$_4$-Partikel sowie der magnetische Gehalt $\mu_{mag}$ der erhaltenen SiO$_2$@Fe$_3$O$_4$-Partikel sind in der nachfolgenden Tabelle wiedergegeben:

| Fe$_3$O$_4$-Partikel [mg] | TEOS [mL] | TEOS [mmol] | EtOH [mL] | Tropfrate [mL·h$^{-1}$] | NH$_4$OH [mL] | P$_{mag}$ [m%] |
|---|---|---|---|---|---|---|
| 120 | 5 | 22,6 | 100 | 26 | 15 | 15,2 |

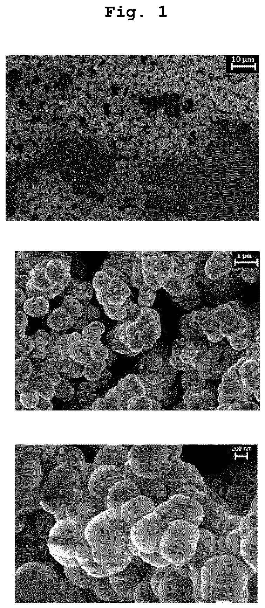

Figur 1 zeigt REM-Aufnahmen der hergestellten SiO$_2$@Fe$_3$O$_4$-Partikel. Die Partikel weisen eine nahezu sphärische Geometrie mit einem durch REM bestimmten mittleren Durchmesser von 699 $\pm$ 112 nm auf.

*ii) Herstellung von TiO$_2$@SiO$_2$@Fe$_3$O$_4$:*

Die SiO$_2$@Fe$_3$O$_4$-Partikel aus dem vorigen Schritt wurden in einem 100 mL Dreihalsrundkolben mit Hilfe eines Ultraschallbads in einer THF/EtOH-Mischung (1:4 v/v) für 5 min dispergiert. Der Dreihalsrundkolben wurde währenddessen an einem KPG-Rührer angeschlossen. Tetrabutylorthotitanat (TBOT) wurde in 20 mL EtOH verdünnt und unter Schutzgas (N$_2$) bei Raumtemperatur langsam (5,0 mL/h) zu der Partikeldispersion getropft. Ammoniak (NH$_4$OH, 25%) und Wasser wurden nach bestimmter Zeit dazugegeben. Nach der Zugabe von TBOT wurde die Partikeldispersion für 1 h weiter gerührt. Anschließend wurden die Partikel fünfmal mit jeweils 15 mL EtOH gereinigt, magnetisch getrennt, unter Vakuum getrocknet und der magnetische Gehalt mit Hilfe von VSM-Messungen bestimmt.

Die eingesetzten Mengen an SiO$_2$@Fe$_3$O$_4$-Partikel, TBOT, EtOH, THF, Wasser und NH$_4$OH sowie der magnetische Gehalt $\mu_{mag}$ der erhaltenen TiO$_2$@SiO$_2$@Fe$_3$O$_4$-Partikel sind in der nachfolgenden Tabelle wiedergegeben:

| Bsp. | SiO$_2$@ Fe$_3$O$_4$ [mg] | TBOT [mL] | THF/ EtOH [ml] | H$_2$O[a] [mL] | NH$_4$OH [μL] | Zeit Zugabe NH$_4$OH[b] [h] | $\mu_{mag}$ [m%] |
|---|---|---|---|---|---|---|---|
| a) | 24 | 0,5 | 25 | 0,5 | 0,1 | 1 | 9,8 |
| b) | 24 | 1 | 25 | 0,5 | 0,1 | 1 | 6,6 |
| c) | 24 | 4 | 25 | 0,5 | 0,1 | 1 | 5,0 |
| d) | 24 | 3 | 25 | 0,25 | 0,05 | 1 | 2,0 |
| e) | 24 | 4 | 25 | 0,25 | 0,05 | 1 | 1,6 |
| f) | 24 | 2 | 25 | 0,25 | 0 | 0 | 1,9 |
| g) | 24 | 2 | 25 | 0,25 | 0,05 | 1 | 7,7 |

(fortgesetzt)

| Bsp. | SiO$_2$@ Fe$_3$O$_4$ [mg] | TBOT [mL] | THF/ EtOH [ml] | H$_2$O$^a$ [mL] | NH$_4$OH [µL] | Zeit Zugabe NH$_4$OH$^b$ [h] | $\mu_{mag}$ [m%] |
|---|---|---|---|---|---|---|---|
| h) | 24 | 2 | 25 | 0,5 | 0,1 | 1 | 4,9 |
| i) | 12 | 1 | 12,5 | 0,25 | 0,025 | 1 | 2,4 |
| j) | 12 | 1 | 12,5 | 0,25 | 0,05 | 1 | 6,2 |
| k) | 12 | 1 | 12,5 | 0,25 | 0,1 | 1 | 4,1 |
| $^a$: H$_2$O wurde eine Stunde nach der Zugabe von NH$_4$OH hinzugefügt. $^b$: Zeit der Zugabe von NH$_4$OH nach dem Beginn der Zugabe von TBOT | | | | | | | |

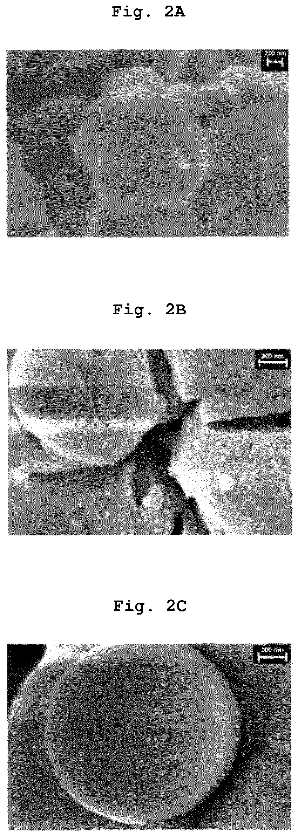

Figur 2 zeigt REM-Aufnahmen der hergestellten TiO$_2$@SiO$_2$@Fe$_3$O$_4$-Partikel, wobei Fig. 2A die in Beispiel 2 f) erhaltenen Partikel, Fig. 2B die in Beispiel 2 h) erhaltenen Partikel und Fig. 2C die in Beispiel 2 d) erhaltenen Partikel zeigt. Es ist zu erkennen, dass eine Durchführung der zweiten Beschichtungsreaktion ohne Zugabe von Base zu einer TiO$_2$-Beschichtung mit einer porösen Morphologie auf der SiO$_2$-Oberfläche führte (Fig. 2A). Die Ergebnisse zeigen außerdem, dass die Bildung von freien TiO$_2$-Partikeln verringert und die Porosität der beschichteten Partikel minimiert werden konnte, wenn der Basenkatalysator erst 60 min nach dem Beginn der Zugabe des TiO$_2$-Vorläufermaterials zugegeben wurde (Fig. 2C).

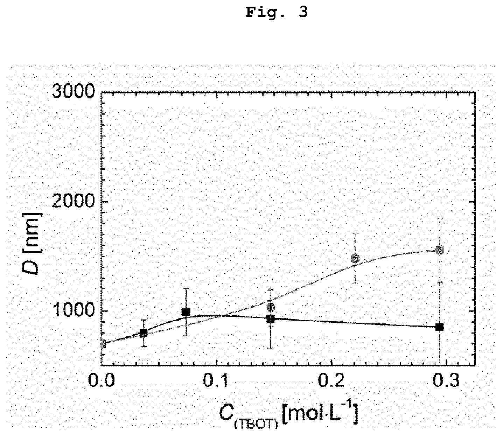

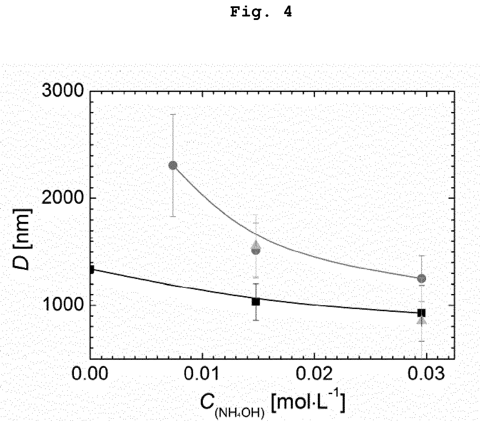

Figur 3 zeigt für zwei verschiedene Basenkonzentrationen (Kreise: 0,014 mol/L NH$_4$OH; Quadrate: 0,029 mol/L) den Einfluss der TBOT-Konzentration auf den Partikeldurchmesser. Figur 4 zeigt für verschiedene TBOT-Konzentrationen (Kreise: 0,07 mol/L TBOT; Quadrate: 0,17 TBOT; Dreiecke: 0,29 mol/L) den Einfluss der NH$_4$OH-Konzentration auf den mittels REM bestimmten Partikeldurchmesser. Sowohl in Figur 3 als auch in Figur 4 bezieht sich die Basenkonzentration auf das Endvolumen der Reaktionsmischung. Die durchgezogenen Linien dienen in beiden Figuren der Veranschaulichung. Eine zunehmende TBOT-Konzentration führte bei moderater Basenkonzentration (0,014 mol/L) zu größeren Durchmessern, während eine Zunahme der Basenkonzentration in kleineren Partikeldurchmessern resultierte. Eine zunehmende TBOT-Konzentration bei hoher Basenkonzentration in der Reaktionsmischung kann zu einer Bildung von freien TiO$_2$-Partikel führen. Eine hohe NH$_4$OH-Konzentration (z.B. 0,029 mol/L) kann aufgrund der schnellen Hydrolose der TBOT-Reste auf der Oberfläche der Partikel zu Bildung von Rissen in den hergestellten Partikeln führen (siehe auch Figur 2B). Bei TBOT-Konzentrationen von 0,17 mol/L - 0,29 mol/L und Basenkonzentrationen von 0,01 mol/L - 0,015 mol/L wurden die besten Beschichtungsergebnisse erhalten (siehe Figur 2C). Die hergestellten Magnetpartikel weisen im Vergleich zu TiO$_2$@Fe$_3$O$_4$-Partikeln, d.h. Fe$_3$O$_4$-Partikeln mit einfacher TiO$_2$-Beschichtung eine farbneutrale optische Erscheinung auf.

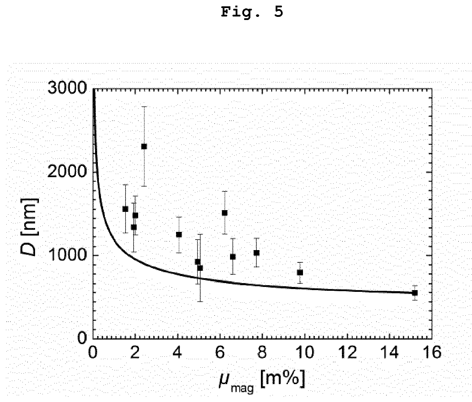

Figur 5 zeigt die Abhängigkeit des Partikeldurchmessers von dem magnetischen Gehalt $\mu_{mag}$ der TiO$_2$@SiO$_2$@Fe$_3$O$_4$-Partikel. Es zeigt sich, dass der magnetische Gehalt der TiO$_2$@SiO$_2$@Fe$_3$O$_4$-Partikel geringer als 15,2%, dem magnetischen Gehalt der als Ausgangsmaterial verwendeten SiO$_2$@Fe$_3$O$_4$-Partikel, ist und mit abnehmendem magnetischen Gehalt die Partikelgröße grundsätzlich zunimmt. Mithin kann auf eine erfolgreiche Beschichtung geschlossen werden. Die durchgezogene Linie stellt den theoretischen Zusammenhang zwischen dem magnetischen Gehalt und dem Partikeldurchmesser dar, der durch folgende Gleichung beschrieben wird:

$$\mu_{\text{mag}} = \frac{m_{(Kern)}}{m_{(kern)} + m_{(Schale)}} = \frac{m_{(Kern)}}{m_{(gesamt)}} = \frac{\rho_{(Kern)} \cdot v_{(Kern)}}{\rho_{(gesamt)} \cdot v_{(gesamt)}} = \frac{\rho_{(Kern)} \cdot d_m^3}{\rho_{(gesamt)} \cdot D^3}$$

,

wobei p: Dichte, m: Masse, $d_m$: Durchmesser Kern und D: Durchmesser Kern-Schale-Partikel, wobei für diesen Fall als Kernpartikel die SiO$_2$@Fe$_3$O$_4$-Partikel gelten und das Ergebnis der obigen Gleichung mit $\mu_{mag}$ in Prozent von SiO$_2$@Fe$_3$O$_4$-Kernpartikel (15,2%) multipliziert werden, um die Gleichung auf die Doppelhüllenarchitektur anzupassen.

**Beispiel 2**

Einbettung von TiO$_2$@SiO$_2$@Fe$_3$O$_4$ in Zahnzement

TiO$_2$@SiO$_2$@Fe$_3$O$_4$-Partikel wurden in Zahnzemente eingebettet, um magnetische Zahnzement-Komposite zu erhalten. Als Zahnzement wurde dualhärtender Zahnzement DC und LC (Variolink Esthetic DC/LC) verwendet. Für die Herstellung der Komposite wurden etwa 0,25 g Zahnzement (LC) in 2 mL EtOH in abgedunkeltem Rollrandglas mit Hilfe eines Ultraschallbads gelöst bzw. dispergiert. $TiO_2@SiO_2@Fe_3O_4$-Partikel wurden dazugegeben und weiterhin mit Hilfe eines Ultraschallbads bei 70 °C für 30 min dispergiert. Das Gemisch wurde im Trockenschrank getrocknet. Der magnetische Gehalt der Komposite wurde anschließend mit Hilfe von VSM-Messungen bestimmt.

Die eingesetzten Mengen an $TiO_2@SiO_2@Fe_3O_4$-Partikel und Zahnzement sowie der magnetische Gehalt $\mu_{mag}$ der eingesetzten Partikel und des erhaltenen Komposits sind in der nachfolgenden Tabelle wiedergegeben:

| Bsp. | $TiO_2@SiO_2@Fe_3O_4$-Partikel [mg] | Zahnzement [mg] | $\mu_{mag}$ Partikel [m%] | $\mu_{mag}$ Komposit [m%] |
|---|---|---|---|---|
| a) | 2,7 | 250 | 1,6 | 0,02 |
| b) | 7,0 | 255 | 1,6 | 0,04 |
| c) | 13,7 | 255 | 1,6 | 0,08 |
| d) | 27,0 | 249 | 1,6 | 0,15 |
| e) | 3,75 | 249 | 2,0 | 0,02 |
| f) | 17,4 | 251 | 2,0 | 0,13 |
| g) | 2,7 | 250 | 7,7 | 0,15 |

Für die in Beispiel 3 untersuchten Prüfkörper wurde das niedrig viskose Komposit vor dem Aushärten zwischen zwei Objektträgern mit je einer Metallscheibe von 300 $\mu$m Dicke als Abstandshalter an jeder Seite gebracht, so dass Prüfkörper von etwa 300 $\mu$m Dicke erhalten wurden. Die Aushärtung erfolgte unter Tageslicht für 6 Stunden.

**Beispiel 3**

Optische Eigenschaften von $TiO_2@SiO_2@Fe_3O_4$ in Zahnzement

Die gemäß Beispiel 2 hergestellten Komposite wurden hinsichtlich ihres optischen Erscheinungsbilds untersucht. Dazu wurde von den Kompositen jeweils ein Foto vor einem schwarz/weiß Hintergrund aufgenommen.

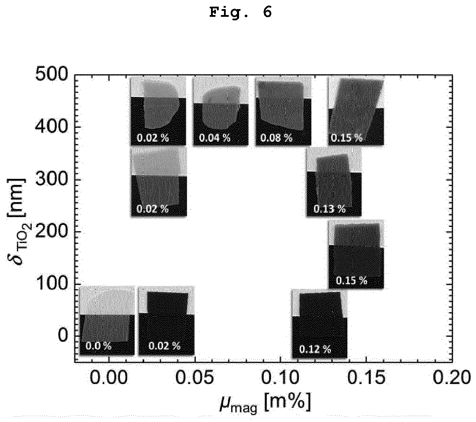

Figur 6 zeigt ein Diagramm mit den dabei erhaltenen Ergebnissen. Dabei ist der magnetische Gehalt $\mu_{mag}$ des Komposits gegen die Schichtdicke der äußeren Hülle $\delta_{TiO2}$ der $TiO_2@SiO_2@Fe_3O_4$-Partikel aufgetragen, wobei die $TiO_2$-Schichtdicke $\delta_{TiO2}$ die Differenz des Durchmessers der doppelhüllenbeschichteten Partikel $TiO_2@SiO_2@Fe_3O_4$ und des Durchmessers der einfach beschichteten Partikel $SiO_2Fe_3O_4$ ist. Dabei stellt die Fotoposition im Diagramm näherungsweise die entsprechenden Koordinaten aus magnetischen Gehalt des Komposits - und damit dem Partikelgehalt des Komposits - und der Hülldicke der Partikel dar.

Zusätzlich zu den sieben Proben aus Beispiel 2 sind in Figur 6 ferner die Fotos von drei Proben dargestellt, die keine $TiO_2@SiO_2@Fe_3O_4$-Partikel enthalten (Schichtdicke der äußeren Hülle $\delta_{TiO2}$ = 0 nm). Von diesen drei Proben weist eine keinerlei Magnetpartikel auf ($\mu_{mag}$ = 0 m%), die anderen beiden enthalten verschiedene Mengen an $SiO_2@Fe_3O_4$-Partikel, um Komposite mit $\mu_{mag}$ = 0,02 m% und 0,12 m% zu ergeben.

Aus Figur 6 ist zu erkennen, dass die Zunahme des magnetischen Gehalts grundsätzlich zu opaken und dunkleren Kompositen führt. Die magnetischen Komposite ohne $TiO_2$-Hülle weisen dabei selbst bei einem magnetischen Gehalt von nur 0,02 m% eine sehr dunkle Farbe auf. Die Bereitstellung einer $TiO_2$-Hülle um die $SiO_2@Fe_3O_4$-Partikel führt hingegen zu einer effektiven Farbabschirmung des magnetischen Kerns der Magnetpartikel. Durch eine Erhöhung der $TiO_2$-Schichtdicke kann die Farbabschirmung des magnetischen Kerns weiter verbessert werden, so dass Komposite mit helleren Farben erhalten werden.

**Vergleichsbeispiel 1**

Herstellung von $Fe_3O_4$-Kernen mit einfacher Beschichtung

In diesem Beispiel wird die Herstellung von $Fe_3O_4$-Kernen mit einer Beschichtung aus polymerisierten 3-(Trimethoxy-silyl)propylmethacrylat (TPM), $ZrO_2$ oder $TiO_2$ beschrieben. Derartig beschichtete Partikel werden im Folgenden auch als $SiO_2$-Acrylat$@Fe_3O_4$, $ZrO_2@Fe_3O_4$ bzw. $TiO_2@Fe_3O_4$ bezeichnet.

*i) Herstellung von $SiO_2$-Acrylat$@Fe_3O_4$:*

Magnetische $Fe_3O_4$-Partikel (würfelförmig; zahlenmittlerer Durchmesser gemäß TEM von 138 $\pm$ 31 nm) wurden in einem 500 mL Dreihalsrundkolben vorgelegt. THF wurde dazugegeben und das Gemisch wurde im Ultraschallbad (100 %, 37 kHz) für 10 min dispergiert. Danach wurde EtOH dazugegeben und das Gemisch im Ultraschallbad (100 %, 37 kHz) für 5 min dispergiert. Anschließend wurde Wasser hinzugefügt und das Gemisch im Ultraschallbad (100 %, 37 kHz) für weitere 5 min dispergiert. Ammoniaklösung (25 %) wurde dazugegeben und das Gemisch wurde im Ultraschallbad (100 %, 37 kHz) für 5 min dispergiert. Anschließend wurde der Dreihalsrundkolben an einen KPG-Rührer (200 U/min) mit einem Rührblatt aus Polytetrafluorethylen angeschlossen. TPM wurde tropfenweise unter Schutzgas (N$_2$) dazugegeben und das Gemisch wurde für 1 h gerührt. Die Polymerisation der Acrylatreste am binären Monomer wurde mit Hilfe von ATRP (Atom Transfer Radical Polymerization) durchgeführt. Die Partikeldispersion wurde für 20 min mit Argon durchgespült. Danach wurde der Katalysator Cu(I)Br dazugegeben und für weitere 20 min mit Argon durchgespült. Der Ligand PMDETA (N,N,N',N',N''-Pentamethyldiethylentriamin) wurde zur Bildung des Katalysator-Ligand-Komplexes hinzugegeben. Nach 20 min Rühren unter Argon bei Raumtemperatur folgte die Zugabe des Initiators MBrP (Methyl-2-brompropionat), wobei das molare Verhältnis von TPM:Cu(I)Br:PMDETA:MBrP 50:1:1:2 betrug. Die Polymerisation wurde unter Schutzgas durchgeführt. Nach 3 h wurden die Partikel fünfmal mit ca. 15 mL EtOH gewaschen und mit einem 400 mT Magnet magnetisch getrennt, um das freie Silica abzutrennen. Danach wurden die freien magnetischen Partikel mit einem ca. 100 mT Magnet magnetisch getrennt. Die Kern-Schale Partikel wurden schließlich unter Vakuum bei 85 °C getrocknet und der magnetische Gehalt mit Hilfe von VSM-Messungen bestimmt.

| Fe$_3$O$_4$-Partikel [mg] | TPM [μL] | CuBr [mg] | THF [mL] | EtOH [mL] | H$_2$O [mL] | NH$_4$OH [mL] | $\mu_{mag}$[a] [m%] |
|---|---|---|---|---|---|---|---|
| 120 | 1200 | 15,8 | 60 | 60 | 240 | 4 | 2,5 |
| [a]: gemessen mit VSM | | | | | | | |

*ii) Herstellung von ZrO$_2$@Fe$_3$O$_4$*

Magnetische Fe$_3$O$_4$-Partikel(würfelförmig; zahlenmittlerer Durchmesser gemäß TEM von 226 ± 77 nm) wurden in einem 100 mL Dreihalsrundkolben mit einem Ultraschallbad für 5 min in 20 mL EtOH dispergiert. Ammoniak (NH$_4$OH, 25%) und 62,5 μl Wasser wurden dazugegeben und für weitere 5 min dispergiert. Der Dreihalsrundkolben wurde währenddessen an einen KPG-Rührer (200 U/min) angeschlossen. Das Ultraschallbad wurde entfernt und mit Hilfe einer Spritzenpumpe wurde bei Raumtemperatur Zirkoniumtetrabutanolat (TBOZ) unter Schutzgas (N$_2$) zu der Partikeldispersion getropft. Nach der Zugabe von TBOZ wurde die Partikeldispersion für eine Stunde gerührt. Anschließend wurden die Partikel fünfmal mit 15 mL EtOH gereinigt und magnetisch getrennt.
Die eingesetzten Mengen an Fe$_3$O$_4$-Partikel, TBOZ, EtOH, Wasser und NH$_4$OH sowie der magnetische Gehalt $\mu_{mag}$ der erhaltenen ZrO$_2$@Fe$_3$O$_4$-Partikel sind in der nachfolgenden Tabelle wiedergegeben:

| Fe$_3$O$_4$-Partikel [mg] | TBOZ [mL] | TBOZ [mmol] | Tropfrate [mL·h$^{-1}$] | H$_2$O [μL] | NH$_4$OH [μL] | $\mu_{mag}$[a] [m%] |
|---|---|---|---|---|---|---|
| 24 | 4 | 11,8 | 5 | 62,5 | 12,5 | 3,8 |
| [a]: gemessen mittels VSM | | | | | | |

*iii) Herstellung von TiO$_2$@Fe$_3$O$_4$*

Magnetische Fe$_3$O$_4$-Partikel(würfelförmig; zahlenmittlerer Durchmesser gemäß TEM von 226 ± 77 nm) wurden in einem 100 mL Dreihalsrundkolben mit einem Ultraschallbad für 5 min in THF/EtOH (1:4 v/v) dispergiert. Ammoniak (NH$_4$OH, 25%) und 500 μL Wasser wurden dazugegeben und für weitere 5 min dispergiert. Mit Hilfe einer Spritzenpumpe wurde in 20 mL EtOH verdünntes TBOT unter Schutzgas (N$_2$) bei Raumtemperatur zu der Partikeldispersion getropft. Nach der Zugabe von TBOT wurde die Partikeldispersion für eine Stunde gerührt. Anschließend wurden die Partikel fünfmal mit jeweils ca. 15 mL EtOH gereinigt, magnetisch getrennt und unter Vakuum getrocknet.
Die eingesetzten Mengen an Fe$_3$O$_4$-Partikel, TBOT, EtOH und NH$_4$OH sowie der magnetische Gehalt $\mu_{mag}$ der erhaltenen TiO$_2$@Fe$_3$O$_4$-Partikel sind in der nachfolgenden Tabelle wiedergegeben:

| Fe$_3$O$_4$-Partikel [mg] | TBOT [mL] | TBOT [mmol] | EtOH/ THF [mL] | Tropfrate [mL·h$^{-1}$] | NH$_4$OH [μL] | $\mu_{mag}$[a] [m%] |
|---|---|---|---|---|---|---|
| 24 | 4 | 11,8 | 25 | 2,5 | 100 | 3,9 |
| [a]: gemessen mittels VSM | | | | | | |

**Vergleichsbeispiel 2**

Optische Eigenschaften von $Fe_3O_4$-Kernen mit einfacher Beschichtung

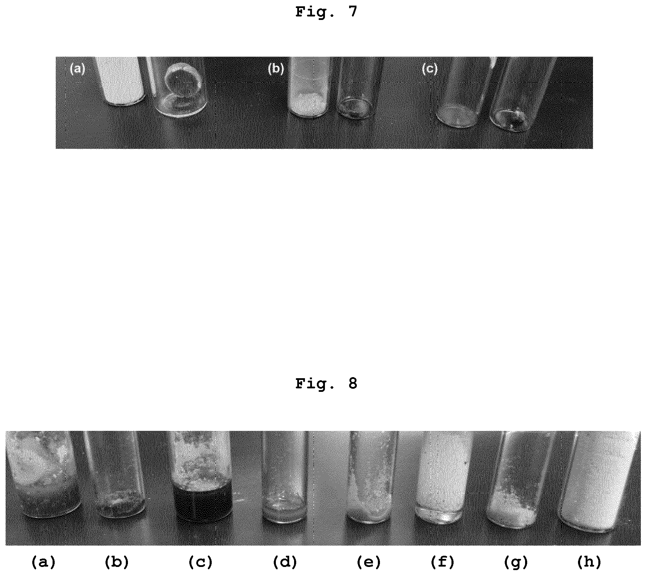

Der Einfluss der verschiedenen Hüllmaterialien auf die optischen Eigenschaften der Magnetpartikel wird in Figur 7 dargestellt. Die Aufnahmen (a) zeigen $SiO_2$-Acrylat@$Fe_3O_4$-Partikel an Luft (links) und $SiO_2$-Acrylat@$Fe_3O_4$-Partikel eingebettet in Zahnzement (rechts). Die Aufnahmen (b) zeigen $ZrO_2$@$Fe_3O_4$-Partikel an Luft (links) und $ZrO_2$@$Fe_3O_4$-Partikel eingebettet in Zahnzement (rechts). Die Aufnahmen (c) zeigen $TiO_2$@$Fe_3O_4$-Partikel an Luft (links) und $TiO_2$@$Fe_3O_4$-Partikel eingebettet in Zahnzement (rechts). Der Brechungsindex der $SiO_2$-Acrylatbeschichtung beträgt etwa 1,5, während $ZrO_2$ einen Brechungsindex von 2,18 und $TiO_2$ einen Brechungsindex von 2,58 aufweisen.

Während $SiO_2$-Acrylat@$Fe_3O_4$-Partikel an Luft eine nahezu neutrale Farbe mit leicht hellgrauem Ton aufwiesen, zeigten $ZrO_2$@$Fe_3O_4$-Partikel an Luft eine graue Farbe und $TiO_2$@$Fe_3O_4$-Partikel an Luft eine nahezu schwarze Farbe.

Anschließend wurde ein Teil der Magnetpartikel in Zahnzement eingebettet. Dazu wurden die beschichteten Magnetpartikel mit einer Konzentration von 1 Gew.-% in Zahnzement (DC) eingebracht und etwa 10 min auf einem Vortex Schüttler bei maximaler Leistung gehalten, bis der Zahnzement fest geworden ist.

Aus Figur 7 ist zu erkennen, dass $SiO_2$-Acrylat@$Fe_3O_4$ in der Zahnzementmatrix eine bedeutend dunklere Farbe als in Kontakt mit Luft zeigte. Diese Beobachtung gilt auch für $ZrO_2$@$Fe_3O_4$ und $TiO_2$@$Fe_3O_4$. Dabei ist zu berücksichtigen, dass der weiße Rand, der auf den Aufnahmen der Zahnzementproben zu beobachten ist, auf die glänzende Oberfläche des Zahnzements und somit auf Reflexionen und nicht auf Effekte der Beschichtung der Magnetpartikel zurückzuführen ist.

Die obigen Beobachtungen zeigen, dass der Brechungsindex-Kontrast zwischen dem Hüllmaterial und der umgebenden Matrix bei dem optischen Erscheinungsbild eine entscheidende Rolle spielt. Dies wird auch in Figur 8 verdeutlicht, die eine Aufnahme von $SiO_2$-Acrylat@$Fe_3O_4$-Partikeln zeigt, die in verschiedenen Medien mit unterschiedlichen Brechungsindizes dispergiert sind, wobei von links nach rechts folgende Medien verwendet sind (jeweils mit Angabe ihres Brechungsindex n): (a) Polystyrol (n = 1,58), (b) o-Xylol (n = 1,50), (c) Polymethylmethacrylat (n = 1,49), (d) 1,4-Dioxan (n = ,42), (e) Ethanol (n = 1,37), (f) Wasser (n = 1,33), (g) Trifluoressigsäure (n = 1,28) und (h) Luft (n = 1,00). Es ist zu erkennen, dass die Farbe der Dispersion mit steigendem Brechungsindex des Lösungsmittels dunkler wird, bis bei einem Brechungsindex von 1,49 ein Maximum erreicht zu werden scheint.

**Beispiel 4**

In diesem Beispiel wird die Analyse von Partiekln mittels VSM-Messungen, TEM-Messungen und REM-Messungen beschrieben.

VSM-Messungen

Die magnetischen Eigenschaften der vorliegend erhaltenen Proben wurden mit Hilfe eines EV7 Vibrating Sample Magnetometers (VSM) der Firma ADE Magnetics untersucht. In Abhängigkeit des angelegten Felds wurde die Magnetisierung einer Probe gemessen, wobei die Probe senkrecht zu einem einheitlichen Magnetisierungsfeld in Schwingung versetzt wurde. Die maximale Feldstärke des verwendeten Elektromagnets (3472-10 GMW) der Firma Magnet Systems betrug 2,1 T. Die Probe wurde einer Schwingungsfrequenz von 75 Hz ausgesetzt und die Hystereseschleifen wurden zwischen $-1,5 \cdot 10^8$ A·m$^{-1}$ und $1,5 \cdot 10^8$ A·m$^{-1}$ aufgenommen.

TEM-Messungen

Die TEM-Messungen wurden an einem Leo 912 Omega der Firma Zeiss durchgeführt. Hierfür wurden die Proben aus einer Partikeldispersion vor den Messungen auf ein kohlenstoffbeschichtetes Kupfergitter getropft und die Proben bei Raumtemperatur getrocknet. Die Bilder wurden mit der Software SiViewer und der Software ImageJ ausgewertet.

REM-Messungen

REM-Aufnahmen wurden mit einem Rasterelektronenmikroskop Zeiss Neon 40 ESB CrossBeam FIB-SEM erzeugt. Die Proben wurden aus einer Partikeldispersion auf ein Silica-Plättchen getropft. Die Proben wurden vor der Messung mit Gold beschichtet. Die Partikelgröße wurde nach der Messung mit der Software ImageJ ausgewertet.

**EP 3 621 089 B1**

**Patentansprüche**

1. Polymerisierbarer Dentalwerkstoff, der mindestens eine radikalisch polymerisierbare Verbindung und Magnetpartikel enthält, die einen magnetischen Kern und eine erste Beschichtung aus erstem Hüllmaterial umfassen, **dadurch gekennzeichnet, dass** auf der vom Kern wegweisenden Oberfläche der ersten Beschichtung eine zweite Beschichtung aus zweitem Hüllmaterial aufgebracht ist, wobei das zweite Hüllmaterial von dem ersten Hüllmaterial verschieden ist und einen höheren Brechungsindex als das erste Hüllmaterial aufweist.

2. Polymerisierbarer Dentalwerkstoff gemäß Anspruch 1, wobei bei den Magnetpartikeln das Verhältnis des Brechungsindex des zweiten Hüllmaterials zu dem Brechungsindex des ersten Hüllmaterials 1,1 bis 2,6, vorzugsweise 1,4 bis 2,0, besonders bevorzugt 1,6 bis 1,8 beträgt.

3. Polymerisierbarer Dentalwerkstoff gemäß einem der vorhergehenden Ansprüche, wobei bei den Magnetpartikeln das Verhältnis der Dicke der zweiten Beschichtung zu der Dicke der ersten Beschichtung 0,1 bis 2,5, vorzugsweise 0,5 bis 2,0, besonders bevorzugt 1,0 bis 2,0 beträgt.

4. Polymerisierbarer Dentalwerkstoff gemäß einem der vorhergehenden Ansprüche, wobei bei den Magnetpartikeln das Verhältnis der Dicke der zweiten Beschichtung zu dem Radius der ersten Beschichtung 0,1 bis 1, vorzugsweise 0,2 bis 0,8, besonders bevorzugt 0,28 bis 0,8 beträgt.

5. Polymerisierbarer Dentalwerkstoff gemäß einem der vorhergehenden Ansprüche, wobei bei den Magnetpartikeln das zweite Hüllmaterial ausgewählt ist aus der Gruppe bestehend aus $TiO_2$, $ZrO_2$, $ZnS$, $Al_2O_3$, Poly (pentabromphenylmethacrylat) und Mischungen davon.

6. Polymerisierbarer Dentalwerkstoff gemäß einem der vorhergehenden Ansprüche, wobei bei den Magnetpartikeln das erste Hüllmaterial ausgewählt ist aus der Gruppe bestehend aus $SiO_2$, $SiO_2$-Acrylat, Polystyrol, Polymethylmethacrylat, Polyvinylalkohol, Polyalkylacrylat, Polycarbonat, $MgF_2$, fluoriertem Polymer, $Al_2O_3$, $ZnO_2$ und Mischungen davon.

7. Polymerisierbarer Dentalwerkstoff gemäß einem der vorhergehenden Ansprüche, wobei bei den Magnetpartikeln das erste Hüllmaterial $SiO_2$ und das zweite Hüllmaterial $TiO_2$ ist.

8. Polymerisierbarer Dentalwerkstoff gemäß einem der vorhergehenden Ansprüche, wobei bei den Magnetpartikeln der magnetische Kern ausgewählt ist aus der Gruppe bestehend aus Eisenoxid, Ferriten der allgemeinen Formel $MeO·Fe_2O_3$, wobei Me ausgewählt ist aus der Gruppe bestehend aus Ni, Zn, Mn, Co, Cu, Mg, Cd, Ba und Sr, Mischferriten der allgemeinen Formel $Me_yFe_{3-y}O_4$, wobei Me ausgewählt ist aus der Gruppe bestehend aus Ni, Zn, Mn, Co, Cu, Mg, Cd, Ba und Sr und y zwischen 0 und 1 beträgt, Metallen und Metalllegierungen aus der Gruppe bestehend aus Co, Ni, Fe, Nd, AlNiCo, FeNi, SmCo und NdFeB sowie Oxiden, insbesondere $CrO_2$.

9. Polymerisierbarer Dentalwerkstoff gemäß einem der vorhergehenden Ansprüche, wobei die Magnetpartikel einen mittleren Durchmesser von 10 bis 2000 nm, vorzugsweise 25 bis 1500 nm, besonders bevorzugt 300 bis 1000 nm aufweisen.

10. Verwendung von Magnetpartikeln umfassend einen magnetischen Kern und eine erste Beschichtung aus erstem Hüllmaterial, wobei auf der vom Kern wegweisenden Oberfläche der ersten Beschichtung eine zweite Beschichtung aus zweitem Hüllmaterial aufgebracht ist, wobei das zweite Hüllmaterial von dem ersten Hüllmaterial verschieden ist und einen höheren Brechungsindex als das erste Hüllmaterial aufweist, zur Herstellung eines polymerisierbaren Dentalwerkstoffs gemäß einem der Ansprüche 1 bis 9.

**Claims**

1. Polymerizable dental material comprising at least one radically polymerizable compound and magnetic particles which comprise a magnetic core and a first coating of a first shell material, **characterized in that** a second coating of a second shell material is applied to the surface of the first coating facing away from the core, wherein the second shell material is different from the first shell material and has a higher refractive index than the first shell material.

2. Polymerizable dental material according to claim 1, wherein, for the magnetic particles, the ratio of the refractive index of the second shell material to the refractive index of the first shell material is from 1.1 to 2.6, preferably 1.4 to 2.0, particularly preferably 1.6 to 1.8.

3. Polymerizable dental material according to any one of the preceding claims, wherein, for the magnetic particles, the ratio of the thickness of the second coating to the thickness of the first coating is from 0.1 to 2.5, preferably 0.5 to 2.0, particularly preferably 1.0 to 2.0.

4. Polymerizable dental material according to any one of the preceding claims, wherein, for the magnetic particles, the ratio of the thickness of the second coating to the radius of the first coating is from 0.1 to 1, preferably 0.2 to 0.8, particularly preferably 0.28 to 0.8.

5. Polymerizable dental material according to any one of the preceding claims, wherein, for the magnetic particles, the second shell material is selected from the group consisting of $TiO_2$, $ZrO_2$, ZnS, $Al_2O_3$, poly (pentabromophenyl methacrylate) and mixtures thereof.

6. Polymerizable dental material according to any one of the preceding claims, wherein, for the magnetic particles, the first shell material is selected from the group consisting of $SiO_2$, $SiO_2$ acrylate, polystyrene, polymethyl methacrylate, polyvinyl alcohol, polyalkyl acrylate, polycarbonate, $MgF_2$, fluorinated polymer, $Al_2O_3$, $ZnO_2$ and mixtures thereof.

7. Polymerizable dental material according to any one of the preceding claims, wherein, for the magnetic particles, the first shell material is $SiO_2$ and the second shell material is $TiO_2$.

8. Polymerizable dental material according to any one of claims, wherein, for the magnetic particles, the magnetic core is selected from the group consisting of iron oxide, ferrites of the general formula $MeO \cdot Fe_2O_3$, wherein Me is selected from the group consisting of Ni, Zn, Mn, Co, Cu, Mg, Cd, Ba and Sr, mixed ferrites of the general formula $Me_yFe_{3-y}O_4$, wherein Me is selected from the group consisting of Ni, Zn, Mn, Co, Cu, Mg, Cd, Ba and Sr, and y is between 0 and 1, metals and metal alloys from the group consisting of Co, Ni, Fe, Nd, AlNiCo, FeNi, SmCo and NdFeB as well as oxides, in particular $CrO_2$.

9. Polymerizable dental material according to any one of the preceding claims, wherin the magnetic particles have an average diameter of from 10 to 2000 nm, preferably 25 to 1500 nm, particularly preferably 300 to 1000 nm.

10. Use of magnetic particles comprising a magnetic core and a first coating of first shell material, wherein a second coating of second shell material is applied to the surface of the first coating facing away from the core, the second shell material being different from the first shell material and having a higher refractive index than the first shell material, for the manufacture of a polymerizable dental material according to any one of claims 1 to 9.

**Revendications**

1. Matériau dentaire polymérisable, qui contient au moins un composé polymérisable par voie radicalaire et des particules magnétiques comprenant un noyau magnétique et un premier revêtement fait d'un premier matériau d'enveloppe, **caractérisé en ce que** sur la surface du premier revêtement éloignée du noyau est disposé un deuxième revêtement fait d'un deuxième matériau d'enveloppe, lequel deuxième matériau d'enveloppe est différent du premier matériau d'enveloppe et présente un indice de réfraction plus élevé que celui du premier matériau d'enveloppe.

2. Matériau dentaire polymérisable conforme à la revendication 1, dans lequel, dans les particules magnétiques, le rapport de l'indice de réfraction du deuxième matériau d'enveloppe à l'indice de réfraction du premier matériau d'enveloppe vaut de 1,1 à 2,6, de préférence de 1,4 à 2,0, et mieux encore de 1,6 à 1,8.

3. Matériau dentaire polymérisable conforme à l'une des revendications précédentes, dans lequel, dans les particules magnétiques, le rapport de l'épaisseur du deuxième revêtement à l'épaisseur du premier revêtement vaut de 0,1 à 2,5, de préférence de 0,5 à 2,0, et mieux encore de 1,0 à 2,0.

4. Matériau dentaire polymérisable conforme à l'une des revendications précédentes, dans lequel, dans les particules magnétiques, le rapport de l'épaisseur du deuxième revêtement au rayon du premier revêtement vaut de 0,1 à 1, de préférence de 0,2 à 0,8, et mieux encore de 0,28 à 0,8.

**5.** Matériau dentaire polymérisable conforme à l'une des revendications précédentes, dans lequel, dans les particules magnétiques, le deuxième matériau d'enveloppe est choisi dans l'ensemble constitué par les $TiO_2$, $ZrO_2$, ZnS, $Al_2O_3$, poly(méthacrylate de pentabromo-phényle), et leurs mélanges.

**6.** Matériau dentaire polymérisable conforme à l'une des revendications précédentes, dans lequel, dans les particules magnétiques, le premier matériau d'enveloppe est choisi dans l'ensemble constitué par les $SiO_2$, $SiO_2$-acrylate, polystyrène, poly(méthacrylate de méthyle), poly(alcool vinylique), poly(acrylate d'alkyle), polycarbonate, $MgF_2$, polymère fluoré, $Al_2O_3$, $ZnO_2$, et leurs mélanges.

**7.** Matériau dentaire polymérisable conforme à l'une des revendications précédentes, dans lequel, dans les particules magnétiques, le premier matériau d'enveloppe est de la $SiO_2$ et le deuxième matériau d'enveloppe est du $TiO_2$.

**8.** Matériau dentaire polymérisable conforme à l'une des revendications précédentes, dans lequel, dans les particules magnétiques, le noyau magnétique est choisi dans l'ensemble constitué par de l'oxyde de fer, les ferrites de formule générale $MeO.Fe_2O_3$ dans laquelle Me est choisi dans l'ensemble constitué par Ni, Zn, Mn, Co, Cu, Mg, Cd, Ba et Sr, les ferrites mixtes de formule générale $Me_y.Fe_{3-y}O_4$ dans laquelle Me est choisi dans l'ensemble constitué par Ni, Zn, Mn, Co, Cu, Mg, Cd, Ba et Sr et l'indice y vaut entre 0 et 1, les métaux et les alliages de métaux de l'ensemble constitué par les Co, Ni, Fe, Nd, Al-Ni-Co, Fe-Ni, Sm-Co et Nd-Fe-B, et les oxydes, en particulier $CrO_2$.

**9.** Matériau dentaire polymérisable conforme à l'une des revendications précédentes, dans lequel les particules magnétiques présentent un diamètre moyen de 10 à 2000 nm, de préférence de 25 à 1500 nm, et mieux encore de 300 à 1000 nm.

**10.** Utilisation de particules magnétiques comprenant un noyau magnétique et un premier revêtement fait d'un premier matériau d'enveloppe, sur la surface duquel premier revêtement éloignée du noyau est disposé un deuxième revêtement fait d'un deuxième matériau d'enveloppe, lequel deuxième matériau d'enveloppe est différent du premier matériau d'enveloppe et présente un indice de réfraction plus élevé que celui du premier matériau d'enveloppe, pour la fabrication d'un matériau dentaire polymérisable conforme à l'une des revendications 1 à 9.

Fig. 1

Fig. 2A

Fig. 2B

Fig. 2C

Fig. 3

Fig. 4

Fig. 5

Fig. 6

Fig. 7

Fig. 8

(a)   (b)   (c)   (d)   (e)   (f)   (g)   (h)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015118816
- DE 4117782
- EP 1894214 A
- WO 2013034777 A
- JP 2000150218 A
- JP 2003002658 A
- US 5194356 A

- WO 2010149150 A
- DE 112012005417
- JP 3032927 A
- EP 0959107 A
- EP 0609897 A
- WO 2016005540 A